Europäisches Patentamt

European Patent Office

Office européen des brevets

(11) Numéro de publication: **0 425 397 A1**

## (12) DEMANDE DE BREVET EUROPEEN

(21) Numéro de dépôt: 90420463.3

(22) Date de dépôt: 26.10.90

(51) Int. Cl.⁵: **C05F 9/02**, **A01K 67/033**, **B65D 88/68**

(30) Priorité: 27.10.89 FR 8914616

(43) Date de publication de la demande:
02.05.91 Bulletin 91/18

(84) Etats contractants désignés:
AT BE CH DE DK ES FR GB GR IT LI LU NL SE

(71) Demandeur: **SOCIETE DE VALORISATION DES DECHETS SOVADEC**
**Roussas**
**F-26230 Grignan(FR)**

(72) Inventeur: **Chareyre, André René**
**Quartier St Andéol**
**F-26160 La Batie Rolland(FR)**

(74) Mandataire: **Maureau, Bernard et al**
**Cabinet GERMAIN & MAUREAU 20, boulevard**
**E. Deruelle B.P. 3011**
**F-69392 Lyon Cédex 03(FR)**

(54) Procédé de traitement par lombricompostage et dispositif pour sa mise en oeuvre.

(57) Ce procédé consiste :
- à introduire, de manière séquentielle et selon une périodicité préétablie, le substrat de déchets organiques par chargement par le haut, à l'intérieur d'au moins un contenant, cylindrique, de section transversale circulaire, polygonale ou autre, d'axe vertical, dont au moins une dimension horizontale transversale est faible, c'est-à-dire de l'ordre de 1 m ou même inférieure, et dont les parois verticales sont au moins partiellement réalisées en un matériau non étanche aux gaz tels que poreux ou aérés,
- à introduire dans cet andain des lombrics qui vont se déplacer ascensionnellement,
- à retirer de manière séquentielle et selon une périodicité préétablie, la couche inférieure de substrat de matière organique préalablement lombricompostée, ce retrait ayant pour effet une descente séquentielle du substrat, par gravité, à une vitesse au plus égale à celle ascensionnelle des lombrics,
- et à évacuer le substrat de matière organique préalablement lombricompostée.
Il est applicable plus particulièrement au domaine du traitement des déchets urbains organiques.

EP 0 425 397 A1

## PROCÉDÉ DE TRAITEMENT PAR LOMBRICOMPOSTAGE ET DISPOSITIF POUR SA MISE EN OEUVRE

La présente invention concerne un procédé de traitement par lombricompostage et un dispositif pour sa mise en oeuvre.

Il est habituel, pour l'obtention d'amendement du type terreau ou compost, à partir de résidus organiques provenant de déchets urbains ou autres similaires, d'avoir recours à des lombrics qui en assurent la pulvérisation, l'aération et le mélange de manière à en favoriser les différentes fermentations aérobies nécessaires à leur transformation en humus.

On connait actuellement un procédé de traitement qui consiste à placer les lombrics dans une couche inférieure et à déposer les résidus organiques en tas ou andains sous forme de plusieurs couches superposées. Ces lombrics, tout en effectuant la pulvérisation, le mélange et l'aération de ces déchets, progressent de manière ascensionnelle vers le haut de cet andain. Une fois qu'ils ont atteint la couche supérieure, cette dernière compostée, est retirée tandis que toutes celles adjacentes, situées en dessous sont évacuées et acheminées en vue de leur stockage et de leur conditionnement.

La couche supérieure, dans laquelle les lombrics se trouvent encore, et qui avait été préalablement enlevée, est alors redéposée à la base d'un nouvel andain, sur lequel sont de nouveau empilées, sous forme stratifiée, plusieurs couches successives de déchets, et le cycle recommence.

Or, ce type de procédé présente l'inconvénient d'une mise en oeuvre de manière discontinue s'opposant de cette façon à un apport régulier de matière organique fraîche à traiter et à l'enlèvement de cette matière organique qui a été lombricompostée.

De plus, ce procédé nécessite une augmentation du nombre de manipulations de la couche supérieure de l'andain, ainsi qu'une constante surveillance de l'évolution de la migration des lombrics à l'intérieur de l'andain, pour pouvoir procéder à l'opération d'enlèvement des différentes couches qui ont été compostées dès que les lombrics ont atteint cette couche supérieure.

De surcroît, la hauteur des andains est limitée à plusieurs décimètres en raison de l'absence d'aération génératrice d'anaérobiose.

On connaît aussi, notamment par le EP.A.91495, un procédé qui consiste à accumuler des déchets organiques dans des tours ou silos, avec chargement par le haut et retrait par le bas au fur et à mesure de la migration ascensionnelle des lombrics. Ce procédé présente, cependant, l'inconvénient de créer des accumulations thermiques considérables, les déchets organiques étant très mauvais conducteurs de la chaleur et ayant une thermogénèse importante résultant de l'activité biologique microbienne et, éventuellement, lombricienne. L'évacuation des calories ainsi engendrées et des gaz résultant de la fermentation et les besoins en oxygène obligent à prévoir des dispositifs complexes de refroidissement, d'échange de fluides dans le substrat et/ou lui imprimant des mouvements. En raison de la complexité des moyens pour sa mise en oeuvre et de leur faible efficacité, ce procédé n'a jamais été exploité industriellement.

La présente invention vise à rémédier à ces inconvénients, en fournissant un procédé de traitement par lombricompostage qui se déroule de manière continue et qui permette un apport séquentiel, et selon une périodicité choisie, des résidus de matière organique à traiter, ainsi qu'un dispositif pour sa mise en oeuvre qui soit simple à réaliser et à utiliser.

A cette fin, le procédé consiste :
- à introduire, de manière séquentielle et selon une périodicité préétablie, le substrat de déchets organiques par chargement par le haut, à l'intérieur d'au moins un contenant, cylindrique, de section transversale circulaire, polygonale ou autre, d'axe vertical, dont au moins une dimension horizontale transversale est faible, c'est-à-dire de l'ordre de 1 m ou même inférieure, et dont les parois verticales sont au moins partiellement réalisées en un matériau non étanche aux gaz tels que poreux ou aérés,
- à introduire dans cet andain des lombrics qui vont se déplacer ascensionnellement,
- à retirer de manière séquentielle et selon une périodicité préétablie, la couche inférieure de substrat de matière organique préalablement lombricompostée, ce retrait ayant pour effet une descente séquentielle du substrat, par gravité, à une vitesse au plus égale à celle ascensionnelle des lombrics,
- et à évacuer le substrat de manière organique préalablement lombricompostée.

Grâce à ce procédé, il n'est plus nécessaire de suivre l'évolution des lombrics à l'intérieur du substrat de matière organique et il n'est plus nécessaire de manipuler la couche supérieure de l'andain. En outre, la faible dimension transversale de chaque contenant facilite l'évacuation des calories et la nature aéré de leurs parois verticales facilitent l'évacuation des gaz de fermentation et l'entrée de l'oxygène.

Grâce à l'établissement d'une périodicité préétablie pour le chargement du substrat de matière organique et le déchargement de celle qui a été préalablement lombricompostée, il est possible, en fonction de la nature des déchets organiques constituant ce substrat à traiter, d'obtenir un rendement maximum.

Suivant une forme d'éxecution générale de l'invention, le dispositif pour la mise en oeuvre du procédé comporte un bâti supérieur fixe supportant au moins un contenant ouvert à son extrémité supérieure et partiellement fermé à son extrémité inférieure, destiné à recevoir le substrat de dechets organiques à traiter, des moyens pour introduire ce substrat de déchets organiques dans le contenant, de manière séquentielle et selon une périodicité prédéterminée, des moyens pour retirer de manière séquentielle la couche inférieure de substrat de matière organique préalablement lombricompostée et des moyens d'évacuation de ce substrat lombricomposté et retiré.

De préférence, ce dispositif comprend plusieurs contenants de forme parallélépipèdique de section transversale rectangulaire aplatie, disposés côte à côte en batterie.

Selon une caractéristique de l'invention, les moyens pour introduire le substrat de dechets organiques à traiter sont constitués par un transporteur à bande dont l'extrémité aval est située au-dessus de l'orifice supérieur de chaque contenant.

Ce type de transporteur est particulièrement bien adapté car il favorise, en fonction du réglage de son débit, l'introduction de manière séquentielle et selon la périodicité préétablie, du substrat de matière organique à traiter.

Avantageusement, les moyens pour évacuer, le substrat de déchets organiques préalablement lombricompostés et retirés, sont constitués par un transporteur à bande dont l'extrémité amont est située en-dessous des moyens pour retirer la couche inférieure du substrat de matière organique à traiter, c'est à dire en dessous de la base de chaque contenant.

Selon une forme de réalisation de l'invention, les parois verticales du contenant sont constituées par du grillage ou autre similaire.

Ce matériau aéré, dont la dimension des ouvertures est adaptée de manière à éviter la perte latérale de matière organique à traiter permet sa bonne aération et améliore de cette façon sa bonne dégradation.

Suivant une première forme d'exécution de ce dispositif, les moyens pour retirer la couche inférieure du substrat de matière organique préalablement lombricompostée, comprennent un bâti supérieur qui supporte le contenant et au-dessous duquel est disposé un bâti inférieur supportant un chariot mobile horizontalement sur une course au moins égale à la longueur de la base du contenant, ce chariot supportant des moyens mécaniques de grattage de la partie du substrat retenu par des barres longitudinales formant grille et obturant partiellement l'orifice inférieur du contenant.

Les barres longitudinales maintiennent normalement le substrat qui ne peut s'écouler entre elles par phénomène de voûtage et de bourrage.

Par exemple, les moyens mécaniques de grattage de la partie inférieure du substrat de matière organique préalablement lombricompostée sont constitués par les pales d'un rotor dont l'arbre horizontal est supporté par le chariot transversalement au bâti fixe, ces pales étant réparties en autant de jeux que l'orifice inférieur du contenant présente d'intervalles entre les barres longitudinales et chaque jeu de pales contenant au moins une paie, des moyens étant prévus pour assurer l'entraînement en rotation du rotor, lors de chaque course horizontale du chariot, à une vitesse de rotation telle que la vitesse linéaire des extrémités libres des pales est différente de celle du chariot.

Le grattage par l'intermédiaire des pales du rotor, supprime le voûtage et le bourrage des déchets du substrat de matière organique préalablement lombricompostée, la couche directement supérieure venant par gravité se bourrer sur les barres longitudinales formant grille.

Suivant une forme d'éxecution simple de l'invention, les moyens d'entraînement du chariot en translation sur sa course longitudinale et d'entraînement en rotation du rotor sont combinés, et sont constitués par un pignon calé à l'une des extrémités de l'arbre de son rotor et engrénant avec une crémaillère longitudinale fixe disposée horizontalement le long de l'un des longerons du bâti supérieur fixe et dont la longueur est au moins égale à celle de la course du chariot, l'arbre du rotor portant à son autre extrémité un second pignon calé sur lequel s'engraine une chaîne de transmission liant en rotation ce pignon au pignon de sortie d'un moto-réducteur, le nombre de dents du pignon engrénant avec la crémaillère fixe étant déterminé de manière à obtenir la différence souhaitée entre la vitesse linéaire du chariot et celle de rotation des extrémités libres des pales du rotor.

Avantageusement, la fréquence de passage du chariot mobile est de 5 semaines et la vitesse en translation est de 4,7 mètres par minute.

Cette fréquence de passage et cette vitesse d'avancement sont fonction de la nature de la matière organique à traiter, ce qui favorise un bon rendement de ce dispositif.

Compte tenu de la longueur généralement importante du contenant, les barres longitudinales doivent être tenues par des traverses intermédiaires dont la présence a pour inconvénient de faire obstacle à la descente d'une partie de la couche inférieure du substrat de matière organique préalablement lombricompostée.

Pour éliminer cet inconvénient, suivant une forme d'éxecution avantageuse de l'invention, d'une part chaque traverse intermédiaire, supportée par les longerons du bâti fixe, de section transversale circulaire est montée pivotante autour de son axe longitudinal qui est parallèle à celui de l'arbre du rotor et d'autre part, elle porte, dans au moins l'un des intervalles entre barre et dans le même plan vertical que le jeu de pales correspondant du rotor du chariot, un jeu de tétons radiaux de dimension telle que l'extrémité libre d'au moins l'un d'eux se trouve sur la trajectoire des pales du jeu de pales précité de manière à être actionné par l'uie d'entre elles lors du passage du chariot et à provoquer de ce fait une rotation sur une fraction de tour de la traverse dont il s'agit.

Il en résulte, évidemment, la chute du substrat de matière organique préalablement lombricompostée et éventuellement retenu sur ces traverses, ce qui évite la formation d'agglomérats ou de zones non grattées.

Avantageusement, chaque palette du rotor comporte un boulon de cisaillement susceptible de rompre sous l'effet d'un effort de 500 Kg et plus.

Il permet de protéger les palettes en cas de blocage.

Suivant une forme avantageuse de cette première variante d'éxecution de l'invention, visant à permettre d'utiliser un seul chariot sous différents contenants, disposés les uns à côté des autres, le bâti inférieur supportant ce dernier est monté de manière déplaçable transversalement sous tous ces contenants.

Par exemple, le bâti inférieur, de même longueur que le bâti supérieur fixe, comporte une glissière de guidage portée par deux flasques dont chacun est supporté, par deux galets de roulement qui permettent un déplacement sous l'ensemble des contenants disposés côte à côte, des moyens moteurs pouvant être prévus pour commander chaque déplacement transversal de ce bâti inférieur de l'un à l'autre des contenants extrêmes.

Selon une variante d'éxecution de l'invention, les barres longitudinales, qui ferment partiellement l'orifice inférieur du contenant, sont constituées par des tiges cylindriques montées pivotantes autour de leur axe longitudinal, au niveau de l'orifice inférieur du contenant considéré, des moyens moteurs étant prévus pour permettre leur entraînement en rotation, de manière séquentielle et selon une périodicité préétablie, par l'intermédiaire de chaines ou courroie et de pignons, et les moyens de grattage, par-dessous la couche inférieure de substrat de matière organique préalablement lombricompostée, sont constitués par des ailettes radiales portées par chaque tige longitudinale, chacune d'elles portant au moins une ailette.

Pour éviter toute rotation non désirée des barres longitudinales et de leurs ailettes, suivant une forme d'exécution simple de cette seconde forme d'exécution de l'invention, il est prévu une crémaillère horizontale portée par des glissières verticales solidaires du bâti fixe, de manière à pouvoir être déplacée entre une position effacée et une position de blocage dans laquelle elle engrène avec les pignons calés à l'une des extrémités des barres longitudinales.

De toute façon, l'invention sera bien comprise à l'aide de la description qui suit en référence au dessin schématique annexé représentant à titre d'exemples non limitatifs deux formes de réalisation du dispositif pour la mise en oeuvre du procédé de lombricompostage selon l'invention.

Figure 1 est une vue en coupe du dispositif suivant la ligne I-I de figure 2.

Figure 2 est une vue en coupe du dispositif suivant la ligne II-II de figure 1.

Figure 3 est une vue en coupe transversale à échelle agrandie d'un jeu de pales du rotor et d'une tige longitudinale.

Figure 4 est une vue en perspective d'une seconde forme de réalisation du dispositif.

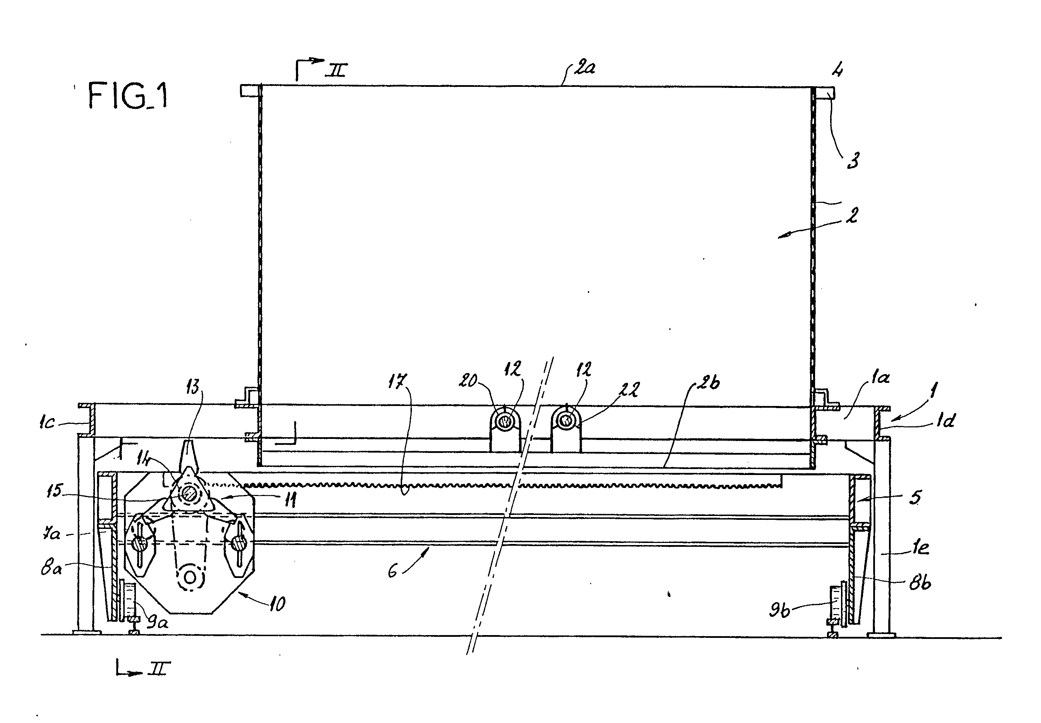

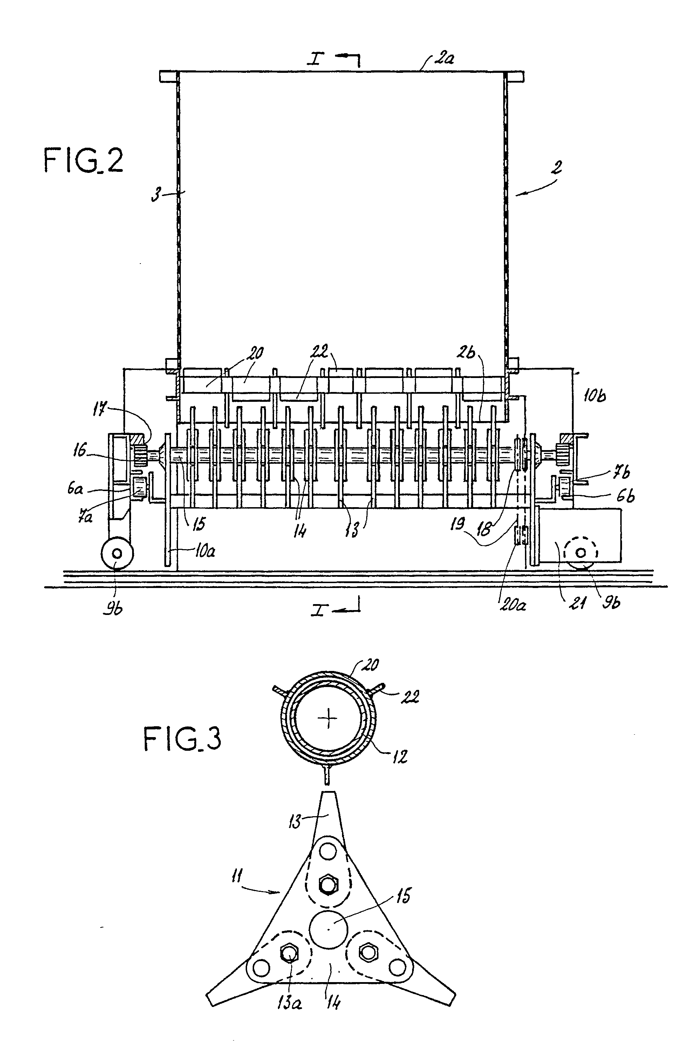

La première forme de réalisation du dispositif selon l'invention, tel qu'il est représenté aux figures 1 et 2, comprend un bâti fixe I constitué par deux longerons 1a et 1b, et deux traverses 1c, 1d disposées parallèlement entre elles et perpendiculairement aux longerons. Ce bâti est surélevé par rapport à la surface du sol par l'intermédiaire de pieds 1e.

Ce bâti supporte un contenant 2, de forme parallèlépipédique, ouvert à son extrémité supérieure 2a et partiellement obturé à son extrémité inférieure 2b. Ce contenant 2 est destiné à recevoir le substrat de matière organique à traiter par son orifice supérieur 2a, de manière séquentielle et selon une périodicité préalablement établie, et à contenir des lombrics.

Les parois verticales 3 de ce contenant 2 sont réalisées en un matériau aéré tel que du grillage. Elles sont surmontées, à leurs extrémités supérieures, par une bordure de renforcement 4 et sont fixées, à leurs extrémités inférieures, par des moyens mécaniques adaptés, sur les longerons et les traverses du bâti supérieur fixe 1.

Sous la base du contenant 2, est disposé un bâti inférieur mobile 5, de même longueur que le bâti fixe supérieur 1 et comprenant une glissière de guidage 6 constituée par deux rails parallèles 6a, 6b. Cette glissière est portée par deux flasques 8a, 8b dont chacun est supporté par deux galets de roulement, respectivement, 9a, 9b. Ces galets permettent la translation de ce bâti mobile 5 sous différents contenants 12 disposés en batterie, côte à côte, afin d'augmenter la surface à gratter. Cette translation peut être réalisée manuellement ou par l'intermédiaire d'un moyen d'entraînement, tel qu'un moteur électrique, non réprésenté sur le dessin.

Sur la glissière de guidage 6 est monté un chariot 10. Ce chariot est donc mobile horizontalement suivant dans une direction perpendiculaire à celle du bâti inférieur mobile 5, sur une course au moins égale à celle de base du contenant 2, et dont une position extrême est représentée à la figure 1. Cette translation horizontale du chariot 10 se fait par l'intermédiaire de galets de roulement 7a, 7b portés par des flasques latéraux 10a, 10b du chariot 10.

Le chariot 10 supporte des moyens mécaniques de grattage 11 de la partie du substrat de matière organique préalablement lombricompostée située au-dessous de l'orifice inférieur 2b du contenant 2, partiellement obturé par des barres longitudinales 12.

Les moyens mécaniques 11 de grattage de cette partie inférieure du substrat de matière organique préalablement lombricompostée sont constitués par les pales 13 d'un rotor 14 dont l'arbre 15 horizontal est supporté par le chariot 10, transversalement au bâti fixe 1. Ces pales 13 sont réparties en autant de jeux que l'orifice inférieur 2a du contenant 2 présente d'intervalles de barres longitudinales 12. Chaque jeu de pales comprend trois pales 13 disposées à 120° l'une de l'autre comme mieux représenté à la figure 3. Chacune de ces pales 13 est bloquée sur son arbre par un boulon de sécurité 13a destiné à rompre lorsque la paie 13 considérée est soumise à un effort tangentiel de l'ordre de 500 kg. La présence de ce boulon évite la rupture de la paie en cas de blocage.

Les moyens d'entraînement du chariot 10 en translation sur sa course longitudinale et d'entraînement en rotation du rotor 14 sont combinés et sont constitués, d'une part, par un pignon câlé 16 à l'une des extrémités de l'arbre de son rotor 14, ce pignon 16 engrenant avec une crémaillère 17 longitudinale fixe, disposée horizontalement le long de l'un des longerons du bâti supérieur fixe 1 et, d'autre part, par un pignon de sortie 21a d'un moto-réducteur 21 lié en rotation, à un troisième pignon 18, câlé sur l'autre extrémité de l'arbre 15 du rotor.

Naturellement, la longueur de cette crémaillère 17 est au moins égale à celle de la course en translation horizontale du chariot mobile 10 de manière à permettre son entraînement sur toute cette longueur.

Le nombre de dents du pignon 16 engrènant avec la crémaillère 17 fixe est déterminé de manière à obtenir une différence de vitesse entre la vitesse linéaire du chariot 10 et celle de la rotation libre des pales 13 du rotor. Les barres longitudinales 12, tenues par des traverses intermédiairès 20, sont uniformément réparties sous la base du contenant 2 au niveau de son orifice inférieur 2b.

Ces traverses 20 ont pour inconvénient de faire obstacle à la descente de la partie inférieure de la couche du substrat de matière organique préalablement lombricompostée. Pour éliminer cet inconvénient, chaque traverse 20 supportée par les longerons la, 1b du bâti fixe 1, de section transversale circulaire est montée pivotante autour de son axe longitudinal qui est parallèle à celui de l'arbre 15 du rotor 14 et porte dans les intervalles entre barre 12 et dans le même plan vertical que le jeu de pales 13, un jeu de tétons 22 radiaux. Chaque jeu de tétons 22, comme représenté à la figure 3, se compose de trois tétons 22 disposés à 120° l'un de l'autre. Ces tétons 22 sont de dimension telle que l'extrémité d'au moins l'un d'eux se trouve sur la trajectoire du jeu de pales 13 correspondant pour pouvoir être actionnée par l'une d'entre elles, lors du passage du chariot 10, et à provoquer, de ce fait, une rotation de la traverse considérée, sur une fraction de tour. Il en résulte la chute du substrat de matière organique préalablement lombricompostée et qui se trouve retenue sur ces traverses 20.

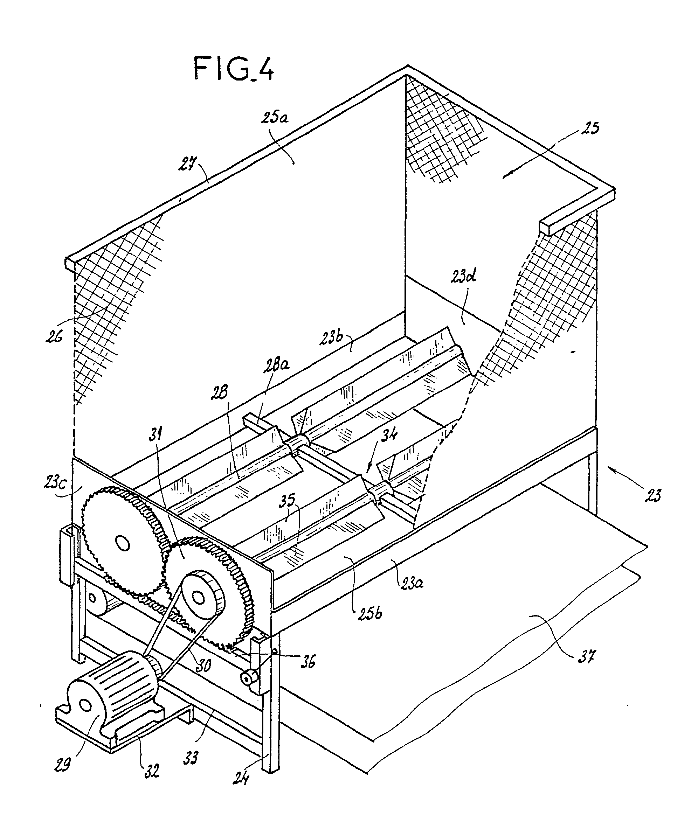

La deuxième forme d'exécution de ce dispositif est représentée plus particulièrement à la figure 4.

Ce dispositif comprend un bâti fixe 23 constitué par deux longerons 23a et 23b et deux plaques latérales de bordures 23c, 23d servant de traverses. Chacune de ces plaques a une hauteur suffisante pour pouvoir supporter des moyens d'entraînement. Ce bâti 23 est surélevé par rapport au sol par l'intermédiaire de pieds 24. Il est supporte un contenant 25 de forme parallèlépipédique ouvert à son extrémité supérieure 25a, et partiellement obturé à son extrémité inférieure 25b. Ce contenant 25 est destiné à recevoir, par son orifice supérieur, le substrat de matières organiques à traiter par des lombrics, de manière séquentielle et selon une périodicité préétablie.

Les parois verticales 26 du contenant 25 sont réalisées en un matériau aéré tel que du grillage. Elles sont surmontées, à leurs extrémités supérieures, par une bordure de protection 27 et sont fixées, à leurs extrémités inférieures, aux longerons 23a, 23b et aux plaques de bordures latérales 23c, 23d.

Des tiges longitudinales 28, qui ferment partiellement l'orifice inférieur 25b du contenant, sont montées pivotantes autour de leur axe longitudinal. En raison de la longueur importante du contenant 25, ces tiges 28 sont supportées, dans leur partie médiane, par des traverses 28a elles-mêmes supportées par les longerons 23a, 23b, et disposées perpendiculairement à l'axe de rotation des tiges 28.

Un moteur 29 est porté par un plateau 32 fixé à une traverse 33 du bâti fixe 23. Ce moteur 29 est destiné à entraîner l'une des deux tiges 28 fermant partiellement l'orifice inférieur du contenant 25 et dont chacune supporte les moyens mécaniques 34 de grattage constitués par quatre ailettes radiales 35 disposées à 90° l'une de l'autre. A cet effet, une poulie 29a, calée sur son arbre de sortie, est liée en rotation, par une courroie 30 à une poulie 3la calée sur l'une des tiges 28, la liaison en rotation entre les deux tiges 28 d'un même contenant étant assurée par deux pignons 31 calés sur elles et engrenant l'un avec l'autre.

Afin d'éviter toute rotation non désirée des barres longitudinales 28, une cremaillère 36 déplaçable horizontalement est disposée sous les pignons 31 de manière à pouvoir être déplacée, entre une position effacée, et une position de blocage dans laquelle elle engrène avec les pignons 31.

Disposé sous la base du contenant 25 un transporteur à bande 37, dont l'extrémité amont est située sous les moyens de grattage permet d'évacuer le substrat de matière organique préalablement lombricompostée et retirée.

Le procédé de lombricompostage se déroule comme décrit ci-dessous :

Le substrat de déchets de matière organique à traiter est introduit de manière séquentielle et selon une périodicité préétablie, par chargement par le haut au niveau de l'orifice supérieur 2a, 25a d'un contenant 2, 25 par l'intermédiaire de moyens mécaniques d'introduction tels qu'un transporteur à bande, non représenté sur le dessin, et dont l'extrémité aval est située sur l'extrémité supérieure du contenant.

Les lombrics sont introduits dans l'andain et vont réaliser leur travail d'aération et de transformation du substrat de manière ascensionnelle.

La couche inférieure de substrat de déchets organiques préalablement lombricompostés est retirée de manière séquentielle et selon une périodicité préétablie. Ce retrait est effectué, dans la première forme de réalisation du dispositif, par des moyens mécaniques de grattage 11 constitués par les pales 13 d'un rotor 14 dont l'arbre 15 horizontal est supporté par le chariot 10 transversalement au bâti fixe 1. Ces pales 13 sont réparties en autant de jeux de pales que l'orifice inférieur 2b du contenant 2 présente d'intervalles entre les barres longitudinales 12 de manière à permettre, grâce à leur rotation, s'accompagnant de la translation du chariot 10 sous la base du contenant 2, l'extraction de la couche inférieure du substrat de déchets de matière organique préalablement lombricompostée.

La matière organique préalablement lombricompostée qui est retenue sur les traverses 20 est quant à elle entraînée, par la rotation d'une fraction de tour de ces traverses montées pivotantes occasionnée par le contact entre les tétons 22 disposés régulièrement à la périphérie des t-averses 20 et de l'une des pales 13 du rotor 14.

Le retrait du substrat de déchets organiques préalablement lombricompostés s'effectue, de manière séquentielle et selon une périodicité préétablie, dans l'autre forme de réalisation du dispositif, par les moyens mécaniques 34 constitués par les quatre ailettes 35 radiales disposées à 90° l'une de l'autre et placées sur les tiges 28 entraînées en rotation par le moteur 29 et obturant partiellement l'orifice inférieur du contenant 25.

L'évacuation du substrat de dechets de matière organique préalablement lombricompostée et retirée est réalisée par l'intermédiaire d'un moyen mécanique tel qu'un transporteur à bande dont l'extrémité amont est située en dessous des moyens mécaniques de grattage c'est à dire en dessous du contenant.

## Revendications

1 - Procédé de traitement par lombricompostage caractérisé en ce qu'il consiste :
- à introduire, de manière séquentielle et selon une périodicité préétablie, le substrat de déchets organiques par chargement par le haut, à l'intérieur d'au moins un contenant, cylindrique, de section transversale circulaire, polygonale ou autre, d'axe vertical, dont au moins une dimension horizontale transversale est faible, c'est-à-dire de l'ordre de 1 m ou même inférieure, et dont les parois verticales sont au moins partiellement réalisées en un matériau non étanche aux gaz tels que poreux ou aérés,
- à introduire dans cet andain des lombrics qui vont se déplacer ascensionnellement,
- à retirer de manière séquentielle et selon une périodicité préétablie, la couche inférieure de substrat de matière organique préalablement lombricompostée, ce retrait ayant pour effet une descente séquentielle du substrat, par gravité, à une vitesse au plus égale à celle ascensionnelle des lombrics,
- et à évacuer le substrat de matière organique préalablement lombricompostée.

2 - Dispositif pour la mise en oeuvre du procédé selon la revendication 1, caractérisé en ce qu'il comporte un bâti supérieur fixe (1) supportant au moins un contenant (2) ouvert à son extrémité supérieure (2a) et partiellement fermé à son extrémité inférieure (2b), destiné à recevoir le substrat de déchets organiques à traiter, des moyens pour introduire ce substrat de déchets organiques dans le contenant (2), de manière séquentielle et selon une périodicité prédéterminée, des moyens pour retirer, de manière séquentielle, la couche inférieure de substrat de matière organique préalablement lombricompostée et des moyens d'évacuation de ce substrat lombricomposté et retiré.

3 - Dispositif selon la revendication 2, caractérisé en ce qu'il comprend plusieurs contenants (2) de forme parallélépipédique de section transversale rectangulaire aplatie, disposés côte à côte en batterie.

4 - Dispositif selon la revendication 2 ou 3, caractérisé en ce que les moyens pour introduire le substrat de déchets organiques à traiter sont constitués par un transporteur a bande dont l'extrémité aval est située au-dessus de l'orifice supérieur (2a) de chaque contenant (2).

5 - Dispositif selon la revendication 2, 3 ou 4, caractérisé en ce que les moyens pour évacuer le substrat de déchets organiques préalablement compostés et retirés sont constitués par un transporteur à bande dont l'extrémité amont est située en-dessous des moyens pour retirer la couche inférieure du substrat de matière organique à traiter, c'est à dire en-dessous de la base de chaque contenant (2).

6 - Dispositif selon l'une quelconque des revendications précédentes, caractérisé en ce que les parois verticales (3) du contenant (2) sont constituées par du grillage ou autre similaire.

7 - Dispositif selon l'une quelconque des revendications précédentes, caractérisé en ce que les moyens pour retirer la couche inférieure des substrats de matière organique préalablement lombricompostée comprennent un bâti supérieur (1) qui supporte un contenant (2) et au-dessous duquel est disposé un bâti inférieur (5) supportant un chariot (10) mobile horizontalement sur une course au moins égale à la longueur de la base du contenant (2), ce chariot (10) supportant des moyens mécaniques de grattage (11) de la partie du substrat retenu au-dessus de l'orifice inférieur (2b) du contenant (2) par des barres longitudinales (12) formant grille.

8 - Dispositif selon la revendication 7, caractérisé en ce que les moyens mécaniques de grattage (11) de la partie inférieure du substrat de matière organique préalablement lombricompostée sont constitués par les pales (13) d'un rotor (14) dont l'arbre (15) horizontal est supporte par le chariot (10) transversalement au bâti fixe (1), ces pales (13) étant réparties en autant de jeux que l'orifice inférieur (2b) du contenant (2) présente d'intervalles entre les barres longitudinales (12) et chaque jeu de pales (13) comportant au moins une pale, des moyens étant prévus pour assurer l'entraînement en rotation du rotor (14) lors de chaque course horizontale du chariot (10) à une vitesse de rotation telle que la vitesse linéaire des extrémités libres des pales (13) soit différente de celle du chariot (10).

9 - Dispositif selon la revendication 8, caractérisé en ce que les moyens d'entraînement du chariot (10) en translation sur sa course longitudinale et d'entraînement en rotation du rotor (14) sont combinés et sont constitués par un pignon calé (16) à l'une des extrémités de l'arbre (15) de son rotor (14) et engrénant avec une cremaillère longitudinale fixe disposée horizontalement le long de l'un des longerons du bâti (1) supérieur fixe et dont la longueur est au moins égale à celle de la course du chariot (10), l'arbre (15) du rotor (14) portant à son autre extrémité un second pignon calé (18) sur lequel s'engrène une chaîne de transmission (19) liant en rotation ce pignon (18) au pignon (21a) de sortie d'un moto-réducteur (21), le nombre de dents du pignon (16) engrénant avec la crémaillère (17) fixe étant déterminé de manière à obtenir la différence souhaitée entre la vitesse linéaire du chariot (10) et celle de rotation des extrémités libres des pales (13) du rotor (14).

10 - Dispositif selon l'une des revendications 7 à 9, caractérisé en ce que les barres longitudinales (12) formant grille au fond de chaque contenant (2) sont tenues par des traverses intermédiaires (20).

11 - Dispositif selon l'ensemble des revendications 8 à 10, caractérisé en ce que, d'une part, chaque traverse intermédiaire (20) supportée par les longerons du bâti fixe (1), de section transversale circulaire est montée pivotante autour de son axe longitudinal qui est parallèle à celui de l'arbre (15) du rotor (14) et d'autre part, elle porte dans au moins l'un des intervalles entre les barres (12) formant grille et dans le même plan vertical que le jeu de pales (13) correspondant du rotor (14) du chariot (10), un jeu de tétons radiaux (22), de dimension telle que l'extrémité libre d'au moins l'un d'eux se trouve sur la trajectoire des pales du jeu de la paie (13) précitée de manière à être actionné par l'une d'entre elles lors du passage du chariot (10) et à provoquer, de ce fait, une rotation, sur une fraction de tour, de la traverse (20) dont il s'agit.

12 - Dispositif selon la revendication 11, caractérisé en ce que chaque pale (13) du rotor (14) comporte un boulon de cisaillement (13a) susceptible de se briser lorsqu'il est soumis à un effort de l'ordre de 500 Kg et plus.

13 - Dispositif selon les revendications 3 et 7, caractérisé en ce que le bâti inférieur (5) de même longueur que le bâti supérieur fixe (1) comporte une glissière de guidage (6) portée par deux flasques (8a,8b) dont chacun est supporté par deux galets de roulement (9a,9b) permettant un déplacement, sous l'ensemble des contenants (2) disposés côte à côte, des moyens moteurs étant prévus pour commander chaque déplacement transversal du bâti inférieur (5)de l'un à l'autre des contenants (2) extrêmes.                        .

14 - Dispositif selon la revendication 2 ou 3, caractérisé en ce que les barres longitudinales qui obturent partiellement l'orifice inférieur de chaque contenant sont constitues par des tiges cylindriques (28) montées pivotantes autour de leur axe longitudinal, au niveau de l'orifice inférieur (25b) du contenant considéré (25), des moyens moteurs (29) étant prévus pour permettre leur entraînement en rotation, de manière séquentielle et selon la périodicité désirée, par l'intermédiaire de chaînes ou de courroies (30) et de pignons (31), et les moyens de grattage (34), par-dessous, de la couche inférieure de substrat de matière organique préalablement lombricompostée sont constitués par des ailettes radiales (35) portées par chaque tige (28), chaque tige (28) portant au moins une ailette (35).

**15** - Dispositif selon la revendication 13, caracterisé en ce qu'une crémaillère horizontale (36) est portée par des glissières verticales solidaires du bâti fixe (23) de manière à pouvoir être déplacée entre une position effacée et une position de blocage dans laquelle elle engrène avec les pignons calés (31) à l'une des extrémités des tiges (28).

FIG_1

EP 0 425 397 A1

FIG.2

FIG.3

# FIG.4

**Office européen
des brevets**

# RAPPORT DE RECHERCHE
## EUROPEENNE

Numéro de la demande

## EP 90 42 0463

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,Y | EP-A-0 091 495  (R. SOLI) <br> * revendications ; figure 1 * <br> − − − | 1-6,14 | C 05 F <br> 9/02 <br> A 01 K 67/033 |
| Y | FR-A-2 482 568  (S. BERNARD ET.AL.) <br> * revendications ; figures 1-4 * <br> − − − | 14 | B 65 D 88/68 |
| Y | FR-A-2 364 875  (D. ROUMENS) <br> * page 4, lignes 3 - 5; revendications ; figures 1, 8 * <br> − − − − − | 14 | |
| | | | **DOMAINES TECHNIQUES RECHERCHES (Int. Cl.5)** <br><br> C 05 F <br> A 01 K <br> B 65 D <br> B 65 G <br> B 07 B |

Le présent rapport de recherche a été établi pour toutes les revendications

| Lieu de la recherche | Date d'achèvement de la recherche | Examinateur |
|---|---|---|
| La Haye | 20 décembre 90 | SCHUT,R.J. |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire
T : théorie ou principe à la base de l'invention

E : document de brevet antérieur, mais publié à la
date de dépôt ou après cette date
D : cité dans la demande
L : cité pour d'autres raisons

&: membre de la même famille, document
correspondant